# EUROPEAN PATENT APPLICATION

(11) **EP 1 344 504 A2**
(43) Date of publication of application: **17.09.2003**
(21) Application number: 03005545.3
(22) Date of filing: 11.03.2003
(51) Int. Cl.: A61F 2/16, B29D 11/02

(54) **Intraocular lens and manufacturing method thereof**

(30) Priority: 11.03.2002 JP 2002065957
(71) Applicant: NIDEK CO., LTD, Gamagori-shi, Aichi 443-0035 (JP)
(72) Inventor: Nagasaka, Shinji, Gamagori-shi, Aichi, 443-0021 (JP); Sunada, Tsutomu, Toyohashi-shi, Aichi, 441-3101 (JP); Oda, Haruo, Gamagori-shi, Aichi, 443-0011 (JP)
(74) Representative: Hofer, Dorothea, Dipl.-Phys.

(57) **Abstract**

An intraocular lens (1) including an optical part (2) having a predetermined refractive power and a support part (3) separately formed from the optical part (2) is characterized in that the optical part (2) is provided in an optical surface thereof with a groove (2a) through which a part of the support part (3) is joined to the optical part (2).

## Description

### BACKGROUND OF THE INVENTION

The present invention relates to an intraocular lens which is placed inside an eye of a patient as a substitute for a crystalline lens of the eye.

Conventionally, an intraocular lens has been known as a substitute for a crystalline lens (lens nucleus) extracted from an eye of a patient in an operative treatment method for cataract. This intraocular lens is inserted by the following steps of first making an incision in an eyeball of the patient's eye; fragmenting and aspirating a clouded crystalline lens (lens nucleus) of the eye through the incision by for example an ultrasonic cataract-surgery device; and then inserting the intraocular lens into the eye through the incision in place of the crystalline lens (lens nucleus). If such incision made for insertion of the intraocular lens is large, it may become a burden on the eyeball of the patient and also cause astigmatism of the patient's eye after the operation. For preventing these disadvantages, it is preferable to make the incision in a minimum size. Therefore, the intraocular lens has been desired to be thinner in thickness.

However, the intraocular lens constructed of an optical part and a support part which are separately molded and integrally combined is produced by the steps of forming a predetermined hole in an outer (circumferential) edge surface of the optical part for attachment of the support part to the optical part, inserting one end of the support part in the hole, and joining the optical part and the support part by adhesive or heat. Accordingly, in the intraocular lens of this construction, it would become more difficult to form the hole as the thickness of the optical part is made thinner. In the conventional intraocular lens of this construction, therefore, the optical part must have a predetermined thickness for allowing formation of the hole.

### SUMMARY OF THE INVENTION

The present invention has been made in view of the above circumstances and has an object to overcome the above problems and to provide an intraocular lens which is constructed so that an optical part and a support part can be joined to each other even if the thickness of the optical part is thinner than conventionally needed and which has a large joining strength, and a manufacturing method of the intraocular lens.

Additional objects and advantages of the invention will be set forth in part in the description which follows and in part will be obvious from the description, or may be learned by practice of the invention. The objects and advantages of the invention may be realized and attained by means of the instrumentalities and combinations particularly pointed out in the appended claims.

Such objects are achieved in accordance with an intraocular lens comprising the features of claim 1, that is, an intraocular lens including: an optical part having a predetermined refractive power; and a support part separately formed from the optical part; characterized in that the optical part is provided in an optical surface thereof with a groove through which a part of the support part is joined to the optical part.

The above objects are also achieved in accordance with a manufacturing method of an intraocular lens comprising the features of claim 6, that is, a manufacturing method of an intraocular lens, characterized by including the steps of: forming a groove in an optical surface of an optical part; and joining a part of a support part to the optical part through the formed groove.

Furthermore, the above objects are also achieved in accordance with a manufacturing method of an intraocular lens comprising the features of claim 8, that is, a manufacturing method of an intraocular lens, characterized by including the steps of disposing a part of a support part in a groove formed in an optical surface of an optical part and applying a polymerizable monomer or prepolymer to the groove and the support part; and polymerizing and curing the applied monomer or prepolymer at a predetermined temperature.

Further developments of the present invention are given in the dependent claims.

### BRIEF DESCRIPTION OF THE DRAWINGS

The accompanying drawings, which are incorporated in and constitute a part of this specification illustrate an embodiment of the invention and, together with the description, serve to explain the objects, advantages and principles of the invention.

In the drawings,
Fig. 1 is a perspective view of an intraocular lens in a preferred embodiment according to the present invention;
Fig. 2A is a plane view of an optical part of the intraocular lens, showing grooves formed therein;
Fig. 2B is a sectional view of the optical part taken along a line A-A in Fig. 2A;
Fig. 2C is a sectional view of the optical part taken along a line B-B in Fig. 2A;
Fig. 3 is a flowchart showing a flow of steps for manufacturing the intraocular lens;
Fig. 4 is a partial plane view of another intraocular lens with a groove having a different outer shape from that shown in Fig. 1; and
Figs. 5A and 5B are partial sectional views of intraocular lenses with grooves having different inner shapes from that shown in Fig. 1.

### DETAILED DESCRIPTION OF THE PREFERRED EMBODIMENTS

A detailed description of a preferred embodiment of an intraocular lens and a manufacturing method thereof embodying the present invention will now be given referring to the accompanying drawings.

Numeral 1 is an intraocular lens constructed of an optical part 2 and a pair of support parts 3. It is to be noted that the number of the support parts may be one or three or more. The optical part 2 can be made of either of a hydrophilic or hydrophobic foldable material and a non-foldable hard material; however, it is preferably made of a foldable material in order to minimize an incision to be formed in a patient's eye. Examples of the hard material include PMMA (polymethyl methacrylate) or the like, while examples of the foldable material include an acrylic material such as HEMA (hydroxymethyl methacrylate) or the like, and silicon, etc. All these materials usable for an intraocular lens are transparent and biocompatibile. The support parts 3 are made of a material having hardness and elasticity so as to be able to hold the optical part 2 within a lens capsule. The material is for example PMMA, polypropylene, polyimide, etc. which are generally used for a support part of an intraocular lens.

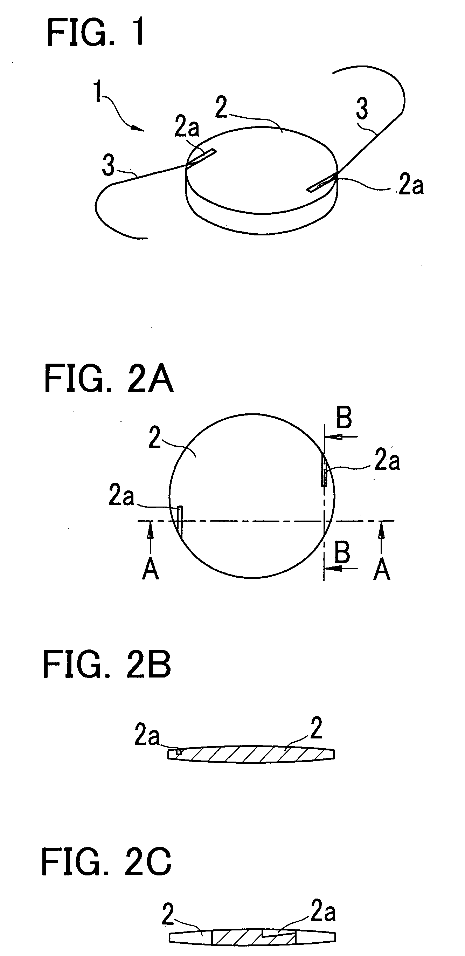

Figs. 2A to 2C are views showing the optical part 2 before the support parts 3 are joined thereto. Specifically, Fig. 2B is a sectional view of the optical part 2 taken along the line A-A in Fig. 2A, and Fig. 2C is a sectional view of the optical part 2 taken along the line B-B in Fig. 2A. As shown in Fig. 2A, one of the surfaces (optical surface) of the optical part 2 is formed with two grooves 2a for attaching and joining the support parts 3 to the optical part 2. Each groove 2a is designed to have a length enough to ensure predetermined joining strength between the optical part 2 and the support part 3 joined thereto. The grooves 2a are formed at positions as far apart as possible from the center of the optical part 2 in order not to restrict patient's vision. The grooves 2a are preferably formed at the positions out of a circle having a diameter of more than 3.0 mm, more preferably 4.0 mm, with the center of the optical part 2 being centered. The width of each groove 2a is preferably equal to or slightly larger than the diameter of the support part 3. In the case where the optical part 2 is made of the foldable material, the width of each groove 2a may be slightly smaller than the diameter of the support part 3.

Furthermore, each groove 2a is formed so that its depth becomes gradually larger (deeper) with a slope from the inlet side of the groove 2a (an outer edge of the optical part 2) toward the inmost side in a longitudinal direction of the groove 2a, as shown in Fig. 2C. This shape of each groove 2a allows the support part 3 to incline frontwards (upwards in Fig. 1) at an angle in the eye when the intraocular lens 1 is placed inside the lens capsule. According to this relation in shape between the optical part 2 and the support parts 3, the optical part 2 can be held and fixed in the lens capsule while keeping the back surface (the lower surface in Figs. 2B and 2C) in contact with the inner back side of the capsule. This makes it possible to prevent the occurrence of after cataract. For the frontward inclination of the support parts 3, the grooves 2a are preferably formed in the front surface (front optical surface) of the optical part 2 (namely, the surface which will be a front side of the optical part 2 placed inside the eye). The grooves 2a may be provided in the surface (optical surface) which will be a back side of the optical part 2; however, in this case, the grooves 2a have to be contrived so as to incline the support parts 3 frontwards.

In the conventional intraocular lens of this construction, the holes for attachment of the support parts are formed in the outer edge of the optical part. In this conventional intraocular lens, as the thickness of the optical part is made thinner, it would become more difficult to form the holes in a sloping shape. In the intraocular lens in the present embodiment, on the other hand, the grooves sloping from the surface (optical surface) side of the optical part 2 toward the inner side thereof are formed instead of the holes in the conventional intraocular lens. Thus, even if the optical part 2 has the thickness thinner than conventionally needed for formation of the holes, the grooves 2a corresponding to the holes can be made. Even when each groove 2a is formed with an uniform depth without a slope, the optical part 2 being thinner than the thickness conventionally needed for formation of the holes can be joined with the support parts 3.

Such grooves 2a can be formed by a cutting process using a drill or the like in the same manner as the formation of the holes in the conventional intraocular lens. Alternatively, the grooves 2a can be formed by laser irradiation using a UV laser such as an excimer laser. In this case, a laser beam is irradiated by a predetermined amount to the front surface (front optical surface) or outer edge surface of the optical part 2, thereby forming each groove 2a in a desired shape. The joining material to join each support part 3 to the optical part 2 may consist of a polymerizable monomer or prepolymer.

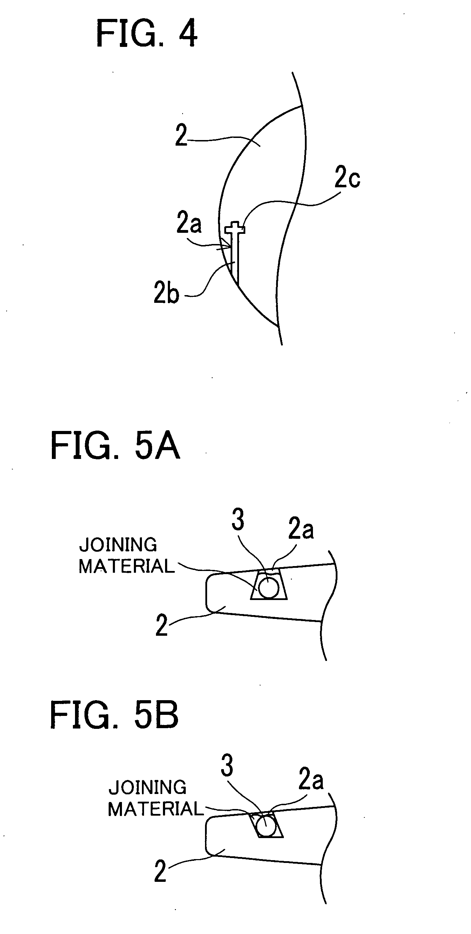

As the entire shape of each groove 2a in plan view, furthermore, various shapes besides the straight shape may be adopted. For example, each groove 2a may be formed in a cross shape constructed of a long groove 2b and a short groove (recess) 2c perpendicularly intersecting with the long groove in the surface (optical surface) of the optical part 2 as shown in Fig. 4. In this case, the support part 3 is preferably provided at its end with a protrusion that can be engaged in the short groove of the cross-shaped groove 2a. This groove 2a having such shape can prevent the support part 3 from coming off together with the joining material from the optical part 2.

As shown in Fig. 5A and 5B, the grooves 2a may be of trapezoidal or parallelogrammatic section in a perpendicular direction to the longitudinal direction of the grooves 2a. These shapes make it possible to prevent the support part 3 joined to the optical part 2 from coming off from the optical part 2 to the upper side (the open side) of the groove 2a. This is because the groove 2a has the sloped wall surfaces (side surfaces) so that the force causing the support part 3 to come off to the upper side of the groove 2a can be restricted by the wall surfaces. This elaborately shaped groove 2a can be produced by use of the above mentioned UV laser beam.

### <Manufacture of the Intraocular lens>

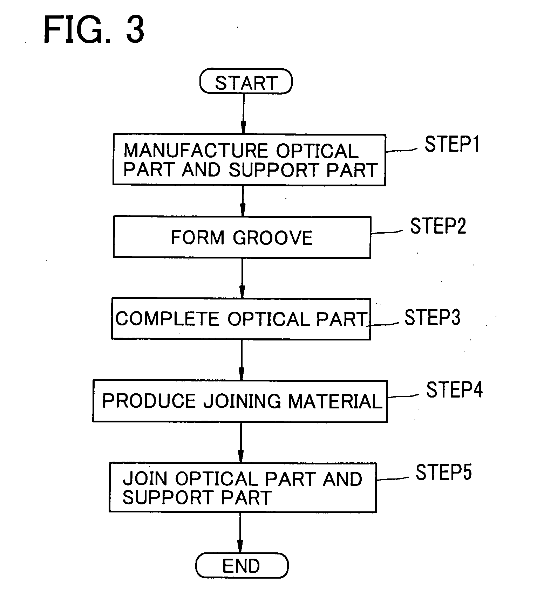

Next, the manufacturing method of the intraocular lens used in the present embodiment is explained with reference to the flowchart of Fig. 3.

At first, a cross-linking agent and a polymerization initiator are added to a material (for example, acrylic material, etc.) for the optical part 2 of the intraocular lens 1, to polymerize and cure the material into a plate shape at a predetermined temperature. The cross-linking agent can be one of any agents conventionally usable for polymerization of materials for the optical part 2, such as 1,4-butanediol di(meth)acrylate, 1,6-hexadiol di(meth)acrylate, or the like. The polymerization initiator can be one of any initiators conventionally usable for manufacture of the optical part 2, such as azoisobutyronitorile and benzoyl peroxide. As needed, ultraviolet absorbent may be included.

The material polymerized and cured in the plate shape is stamped into a circular disk shape. One surface of this disk (which is a front side when the optical part 2 is placed inside the eye) is processed in a cutting process so as to have a predetermined refractive power. On the other hand, a wire rod made of a material for the support part 3 such as polymethylmetacrylate or the like is formed into a loop shape and is cut at a predetermined length, thereby completing formation of the support part 3 (Step 1). Although the outer shape of the optical part 2 is formed in the cutting process in the present embodiment, instead thereof, a method of pouring a base material into a mold to form the optical part 2 may be used.

Subsequently, blocking of the optical part 2 produced in Step 1 is performed, a manual drill is operated in contact with the surface (optical surface) of the optical part 2 (on which the cutting process was made) to form grooves 2a with a predetermined width and depth (Step 2). After this formation of the grooves 2a, deblocking of the optical part 2 is performed and the optical part 2 is turned over. With the other surface unprocessed in the cutting process being upward, the optical part 2 is applied with blocking again and then that surface is cut so as to have predetermined refractive power, thereby completing formation of the optical part 2 (Step 3).

Next, the joining material is prepared by using a monomer or prepolymer of the same kind as the material for the optical part 2. In the case of the monomer, it is sufficient only to add the polymerization initiator to the material (monomer) for the optical part 2. In the case of the prepolymer, on the other hand, it is necessary to add the polymerization initiator to the material (monomer) for the optical part 2 and heat the mixture at a predetermined temperature while agitating, so that the prepolymer is used in an advanced state of polymerization reaction to a certain degree (Step 4).

After that, the support parts 3 are set in the grooves 2a of the optical part 2 and the joining material produced in Step 4 is applied to the grooves 2a (the support parts 3) by use of a syringe or the like. The intraocular lens 1 with the joining material applied is put in an oven that is set to a predetermined temperature (in a range of about 60°C to about 90°C) to heat the intraocular lens 1 for 24 hours, thereby polymerizing the joining material. Thus, the optical part 2 and the support parts 3 are joined, resulting in the finished intraocular lens 1 (Step 5).

Although the application of the joining material is performed by using the syringe or the like in the present embodiment, another manner may be adopted. For instance, a base end portion of the support part 3 is dipped in the joining material and then put in the groove 2a, and similarly heated for polymerization and cure of the joining material.

### <Evaluations of joining strength>

The evaluations of the joining strength between the optical part 2 and the support parts 3 in the intraocular lens 1 in the present embodiment were made using various kinds of joining materials and different polymerizing conditions.

### <Example 1>

In the first example, the base material monomer for the optical part 2 was prepared by mixing ethylene glycol phenyl ether acrylate (EGPEA) of 162g., n-butyl methacrylate of 119.1g., n-butyl acrylate of 12g., 1,4-butanediol dimethacrylate of 6g. as the cross-linking agent, azoisobutyronitorile of 0.3g. as the polymerization initiator, and the ultraviolet absorbent (manufactured by CB Reserch and Development Inc., UV-X) of 0.9g., respectively.

This base material monomer is poured into a mold for a plate shape. The mold is then placed in a constant temperature water bath controlled at temperatures in a range of about 50°C to about 70°C, causing polymerization for about 24 hours. Subsequently, the mold is put in an oven heated at temperatures in a range of about 80°C to about 100°C to cause polymerization for about 24 hours. Then, the mold is taken out of the oven and placed in a vacuum oven to cause further reaction at about 100°C for about 24 hours, completing the polymerization. As a result, an acrylic soft base material was produced.

The acrylic soft base material was frozen and stamped into a disk of 5.5 mm in diameter and 1 mm in thickness. This disk (optical part 2) was machined by means of the drill operated in contact with the surface of the disk to form a rectangular groove 2a of 0.15 mm in width and 0.15 mm in depth. Further, a wire rod made of polymethylmetacrylate (0.15 mm in diameter) is formed into a loop shape and cut at a predetermined length, producing the support part 3.

The support part 3 was partially set in the groove 2a. The joining material made of the base material monomer mentioned above was applied so as to cover the entire joining portion of the support part 3 by use of the syringe. During the application, the joining material constructed of a monomer ran off at once from a needlepoint of the syringe. Thus, the operability was unsatisfactory.

After the application of the joining material, the disk was put in an air oven and heated at 60°C for 24 hours, completing the joining of the support part 3 to the disk. Six pieces each constructed of a disk and support parts 3 joined thereto in that manner were produced. In addition, other six pieces each constructed of a disk and support parts 3 joined thereto in the same manner but at 90°C for 24 hours were produced.

Each disk joined with each support part 3 obtained in the above manner was tensioned by a tensile tester (manufactured by Chatillon, LTC and LTCM-3) to measure a tensile strength between the disk and the support part 3. A measuring method was made based on ISO 11979-3 (Intraocular lens standard, Measurement of tensile strength of a support part). The results are shown in Table 1.

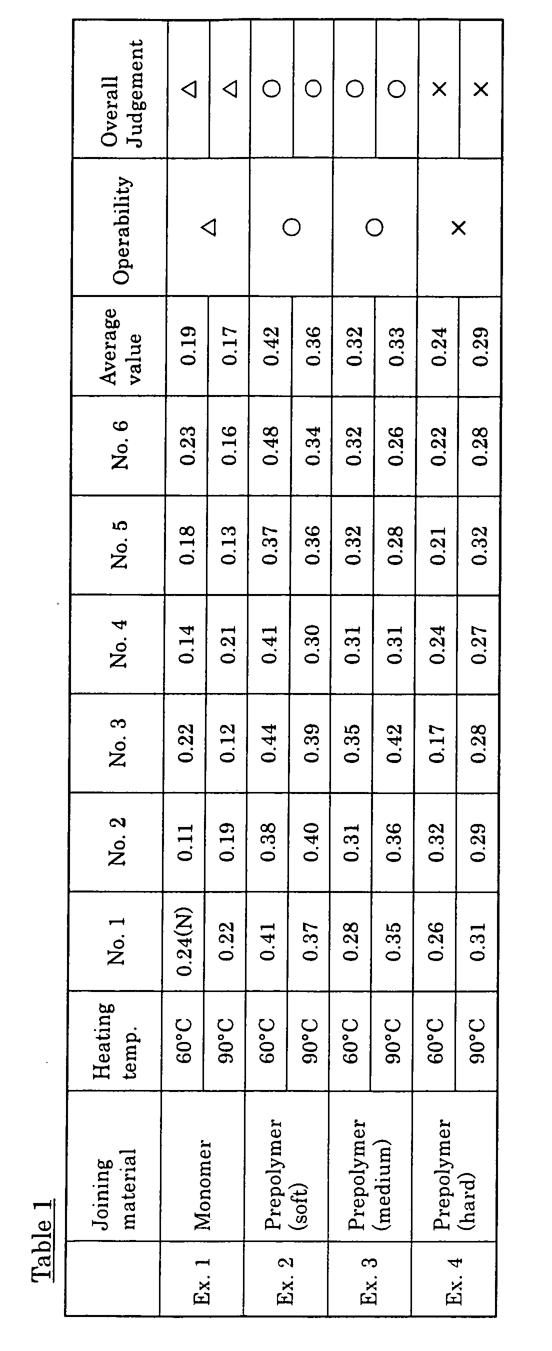

### <Example 2>

Identical disks (optical parts 2) and support parts 3 to those in the example 1 were used. A joining material consisting of a prepolymer was used, which was prepared according to the following steps.

The base material monomer used in the example 1, of about 20 ml, was poured in a 50 ml screw tube, and a stirring bar was put therein and a cap was attached thereto. A hot-stirrer was arranged so that the temperature of water in a beaker set in the hot-stirrer became steady at 70°C. The screw tube was set so that the liquid level of the monomer in the tube was completely submerged in the water in the beaker, and then the monomer was stirred (at a stirring speed of 500 rpm). This stir was continued until the monomer had such a consistency that the liquid surface changed from a cone-shaped swirl state into a flat state. As soon as the liquid surface became apparently flat, the screw tube was taken out of the beaker and put in water in a pot holding therein a cold insulator, thereby cooling the monomer. Simultaneously, the monomer was stirred at high speed by means of the stirrer so as not to cure any more. Thus, the prepolymer (soft) was produced.

The produced prepolymer (soft) was used to join each disk and each support part 3 in the same steps as in the example 1. Six joined pieces were made at each polymerizing temperature of 60°C and 90°C. The application of the joining material by means of the syringe was made with good operability because the joining material had a certain degree of consistency.

Each piece constructed of the disk and the support parts 3 was tensioned by the tensile tester to measure the tensile strength between the disk and the support parts 3 in each piece. The results are shown in Table 1.

### <Example 3>

Identical disks (optical parts 2) and support parts 3 to those in the example 1 were used. A joining material consisting of a prepolymer was used, which was made in the same procedures as in the example 2, except for the condition that the screw tube was taken out of the beaker after a lapse of about 15 seconds from the time the liquid surface of the monomer became flat. The monomer was stirred while being cooled so as not to cure any more. Thus, the prepolymer (medium) was produced.

The produced prepolymer (medium) was used to join the disks and the support parts 3 in the same steps as in the example 1. Six joined pieces were produced at each polymerizing temperature of 60°C and 90°C. The application of the joining material by means of the syringe was made with good operability because the joining material had a certain degree of consistency.

Each piece constructed of the disk and the support parts 3 was tensioned by the tensile tester to measure the tensile strength between the disk and the support parts 3 in each piece. The results are shown in Table 1.

### <Example 4>

Identical disks (optical parts 2) and support parts 3 to those in the example 1 were used. A joining material consisting of a prepolymer was used, which was made in the same procedures as in the example 2, except for the condition that the screw tube was taken out of the beaker after a lapse of about 30 seconds from the time the liquid surface of the monomer became flat. The monomer was stirred while being cooled so as not to cure any more. Thus, the prepolymer (hard) was yielded.

The produced prepolymer (hard) was used to join the disks and the support parts 3 in the same steps as in the example 1. Six joined pieces were produced at each polymerizing temperature of 60°C and 90°C. The application of the joining material by means of the syringe was made with poor operability because the joining material had so high consistency as to form sticky threads at the needlepoint.

Each piece constructed of the disk and the support parts 3 was tensioned by the tensile tester to measure the tensile strength between the disk and the support parts 3 in each piece. The results are shown in Table 1.

As shown in Table 1, differences in tensile strength according to the heating temperatures (60°C and 90°C) were not much found, but differences in tensile strength according to the joining materials (the examples 1 to 4) were found. In the case of the joining material consisting of a monomer (the example 1), as the reason that the tensile strength was low, it is conceivable that this material, having no polymer chain, is apt to be easily saturated in the optical part 2 (the disk in the example), so that the amount of the joining material remaining in the groove 2a is decreased. In the case of the joining material consisting of a prepolymer (hard) (the example 4), on the other hand, it is conceivable that this material is hard to be saturated in the optical part 2 due to having polymer chains, but the material is in a further advanced state of polymerization reaction as compared with other joining materials, so that the joining force of this material is inferior to those of the prepolymer (soft) and the prepolymer (medium).

With respect to the operability of the joining materials, in the case of applying the joining materials through the needlepoint of for example a syringe by utilizing a capillary phenomenon thereof, the material having higher consistency such as a prepolymer is easy to apply because the amount of application can be controlled partly according to how the needlepoint is in contact with the support part 3. However, the prepolymer having high consistency such as the prepolymer (hard) would form sticky threads at the needlepoint and therefore be difficult to apply. If the joining material has too low consistency, such as the monomer, the material would run off at once at the moment that the needlepoint is brought into contact with the support part 3. This kind of joining material is difficult to apply. In this view, to apply the joining material by use of the needlepoint or the like, the prepolymer (soft) and the prepolymer (medium) are preferably used because of their appropriate consistency and operability.

Furthermore, the support part 3 must have a tensile strength of 0.25N in conformity to a standard intraocular lens in an International Organization for Standardization (ISO). The joining material consisting of a monomer therefore fails to meet this standard. However, if the groove 2a is designed so as to have the width, length, shape, etc. as shown in Figs. 4 and 5, even the joining material consisting of a monomer can have the predetermined tensile strength.

According to the present invention, as mentioned above, the thickness of the optical part can be thinner as compared with those of conventional intraocular lenses, while having a joining strength needed as an intraocular lens, so that an incision to be made in a patient's eye for insertion of the intraocular lens can be reduced in size.

While the presently preferred embodiment of the present invention has been shown and described, it is to be understood that this disclosure is for the purpose of illustration and that various changes and modifications may be made without departing from the scope of the invention as set forth in the appended claims.

## Claims

1. An intraocular lens (1) including:
an optical part (2) having a predetermined refractive power; and
a support part (3) separately formed from the optical part (2) ;
**characterized in that** the optical part (2) is provieded in an optical surface thereof with a groove (2a) through which a part of the support part (3) is joined to the optical part (2).

2. The intraocular lens according to claim 1, wherein the groove (2a) is formed having a depth that becomes gradually larger from an outer edge of the optical part (2) in a longitudinal direction of the groove.

3. The intraocular lens according to claim 1 or 2, wherein the groove (2a) is formed in trapezoidal or parallelogrammatic section in a perpendicular direction to a longitudinal direction of the groove.

4. The intraocular lens according to one of claims 1 to 3, wherein the support part (3) is provided with a protrusion at a portion to be disposed in the groove (2a), and the groove (2a) is provided with a recess which engages the protrusion.

5. The intraocular lens according to one of claims 1 to 4, wherein the part of the support part and the optical part are joined through the groove by use of a joining material consisting of a polymerizable monomer or prepolymer.

6. A manufacturing method of an intraocular lens, **characterized by** including the steps of:
forming a groove in an optical surface of an optical part; and
joining a part of a support part to the optical part through the formed groove.

7. The manufacturing method according to claim 6, wherein the joining step includes the steps of:
disposing the part of the support part in the formed groove and applying a polymerizable monomer or prepolymer to the groove and the support part; and
polymerizing and curing the applied monomer or prepolymer at a predetermined temperature.

8. A manufacturing method of an intraocular lens, **characterized by** including the steps of:
disposing a part of a support part in a groove formed in an optical surface of an optical part and applying a polymerizable monomer or prepolymer to the groove and the support part; and
polymerizing and curing the applied monomer or prepolymer at a predetermined temperature.
